# EUROPEAN PATENT APPLICATION

(11) **EP 1 609 444 A1**
(43) Date of publication of application: **28.12.2005**
(21) Application number: 04027688.3
(22) Date of filing: 22.11.2004
(51) Int. Cl.: A61F 2/44, A61B 17/70

(54) **Implant capable of being implanted through a pedicle for vertebral body reconstruction**

(30) Priority: 21.06.2004 CN 200420066877
(71) Applicant: Kung-Chia, Li, Taipei City, Taiwan (CN)
(72) Inventor: Kung-Chia, Li, Taipei City, Taiwan (CN)
(74) Representative: Kador & Partner

(57) **Abstract**

A body augmenter (1) capable of being planted through a pedicle (21) for vertebral body reconstruction,
the body augmenter (1) having a substantially rectangular bar-shape; the body augmenter (1) having a first end, a second end (14) in the opposite direction of the first end, and four sides (11); the body augmenter (1) having a recess (15) on the second end (14), and a screw hole (16) lengthwise formed on the second end (14) and communicating with the recess (15); each of the four sides (11) having a sloping portion (12) near to the first end; the four sides (11) of the body augmenter (1) having cavities (13) on other portions thereof, which cavities (13) communicate with each other, and form a hollow area together.

## Description

### BACKGROUND OF THE INVENTION

### 1. Field of the invention

The present invention relates to a body augmenter capable of being planted through a pedicle for vertebral body reconstruction, more particularly, which is used for treatment of vertebral burst fractures, and vertebral compression fractures.

### 2. Brief Description of the Prior Art

Vertebral body fractures including acute burst fracture or chronic compression fracture with kyphosis were treated in various ways. Acute burst fracture can be treated conservatively, surgical posterior instrumentation, or even anterior bone graft and posterior instrumentation. However, conservative treatment is limited to those with neural intact, non-osteoporosis, good compliance to wear brace and less kyphosis. Posterior instrumentation alone is easy and simple but may lead to 20% implant failure. However, combining anterior and posterior approaches proved to have mechanical advantages, but surgery itself is a suffering to elder patients. The best way to those not good for conservative treatment will be posterior approach with functions of anterior approach simultaneously.

In chronic osteoporotic compression fractures may induce lots of morbilities including chronic back pain, GI dysfunction, decreased pulmonary vital capacity, and feasible new compression fractures. The various methods including surgical instrumentation, vertebral augmentation with cement, kyphoplasty etc. The techniques improved and results become better recently. However, instrumentation may induce decreased spine ROM, implant failure, and adjacent segment disease. The cement either in augmentation or kyphoplasty may induce cement disease. The kyphosis correction is not perfect in kyphoplasty especially the chronic cases with anterior partial or fully fusion. The body augmenter tried to correct the collapsed vertebral body and lead to union with bone graft either combined short posterior instrumentation or cement techniques.

### SUMMARY OF THE INVENTION

It is a main object of the present invention to provide a body augmenter, which can be planted into an osteoporosis-effected vertebra from a pedicle of the vertebra, for helping maintain or restore normal position and size of the vertebra.

Through the vertebral pedicle, a device, fit the pedicle anatomy, was created to reconstruct the fractured or collapsed vertebral body. The device has a substantially rectangular bar-shape, and has a first end, a second end, four sides, a recess on the second end, and a screw hole; each of the four sides having a sloping portion near to the first end; the four sides of the body augmenter having cavities on other portions thereof, which cavities communicate with each other to form a hollow area together. After the body augmenter is planted in a vertebra, harvested autogenous bone or artificial bone is injected into the vertebra such that both the hollow area of the body augmenter and the space between the body augmenter and the vertebra are filled with the injected bone, and the body augmenter is securely fixed in the planting hole. The fractured body was reduced and maintained by such device until the bone growth and fracture union.

### BRIEF DESCRIPTION OF THE DRAWINGS

The present invention will be better understood by referring to the accompanying drawings, wherein:
Fig. 1 is a perspective view of the body augmenter for vertebral body reconstruction according to the present invention,
Fig. 2 is a cross-sectional view of the body augmenter for vertebral body reconstruction in the present invention in use, and
Fig. 3 is a cross-sectional view of the body augmenter for vertebral body reconstruction in the present invention, used in the second way.

### DETAILED DESCRIPTION OF THE PREFERRED EMBODIMENTS

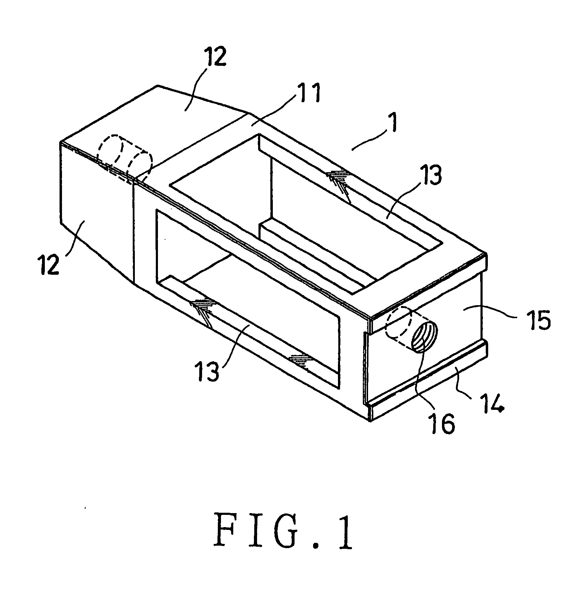

Referring to Fig. 1, a preferred embodiment of a body augmenter 1 for vertebral body reconstruction in the present invention is substantially rectangular bar-shaped, and has a first end, a second end 14 in the opposite direction of the first end, four long sides 11, sloping portions 12 at those portions of the four long sides 11 that is near to the first end, cavities 13 on other portions of the four long sides 11, a recess 15 on the second 14 end, and a screw hole 16 lengthwise extending on the second end 14; the cavities 13 communicate with each other to form a hollow area together; the screw hole 16 communicates with the hollow area as well as the recess 15. The present body augmenter 1 is provided particularly for reconstruction of a collapsed vertebra that is still relatively complete in structure, and doesn't need adjustment.

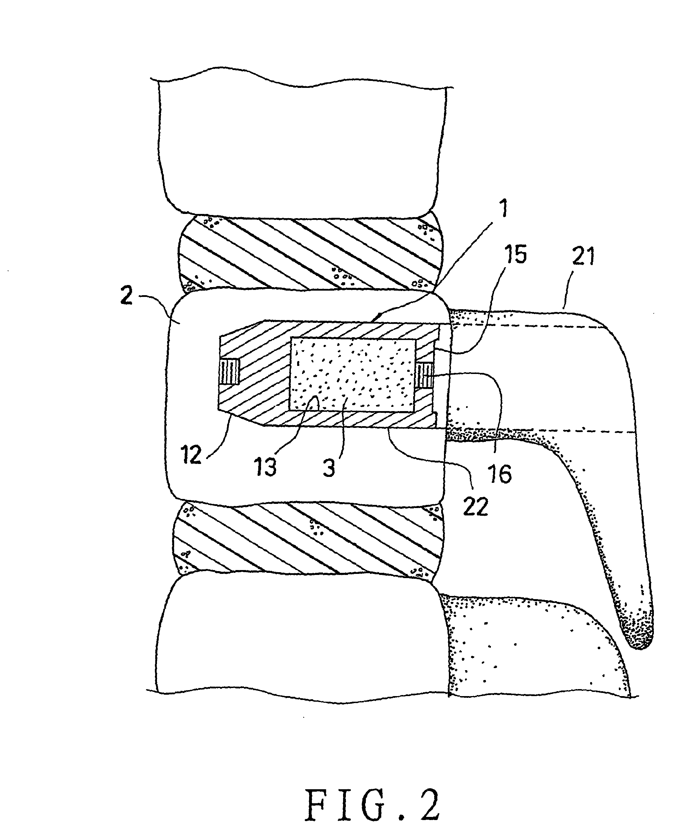

Referring to Fig. 2, to plant the body augmenter 1 into a vertebra 2, first a planting hole 22 is formed, which extends through a pedicle 21 of the vertebra 2 and into the main body of the vertebra 2, i.e. the vertebral body, and which is substantially as big as the body augmenter 1. Second, a planting tool (not shown) is screwed into the screw hole 16 of the body augmenter 1 at front end, and then the body augmenter 1 is planted into the planting hole 22 in the vertebral body with the help of the planting tool, and the planting tool is removed from the body augmenter 1. Finally, harvested autogenous bone or artificial bone is injected into the vertebra 2 through the planting hole 22 such that both the hollow area of the body augmenter 1 and the space between the body augmenter 1 and the vertebra 2 are filled with the injected bone, and the body augmenter 1 is securely fixed in the planting hole 22.

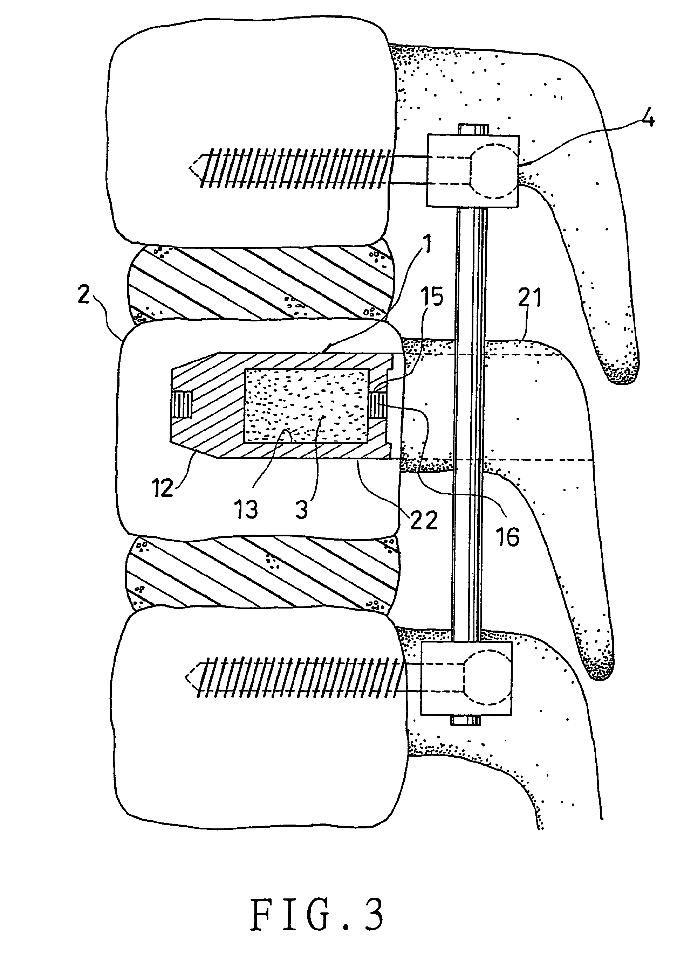

Referring to Fig. 3, if it is necessary, a pedicle screw system 4 can be applied to both of vertebras next to the vertebra 2 for helping increase stability of the spine after planting of the body augmenter 1 of the present invention.

From the above description, it can be easily understood that the body augmenter of the present invention has advantages as followings:
1. The body augmenter is planted into a collapsed vertebra from a pedicle of the vertebra instead of via the thoracic or abdominal cavity of the patient therefore the operation is relatively safe, and mechanical strength of the vertebra is increased.
2. The body augmenter can be used alone on a collapsed vertebra without the need for operation being done on other healthy vertebras near to the collapsed vertebra, which operation will cause the other healthy vertebras to lose the freedom to move.
3. Because the body augmenter is formed with a hollow area, after the planting hole 22 and the hollow area is filled with harvested autogenous bone graft or artificial bone, sponge bone of the vertebra and the harvested autogenous bone graft (or artificial bone) will grow and connect together, and the body augmenter will be more firmly connected with the vertebra.
4. There is no need for laminectomy surgery in planting the present body augmenter because the present body augmenter is planted through the pedicle.

## Claims

1. A body augmenter 1 capable of being planted through a pedicle 21 for vertebral body reconstruction,
the body augmenter 1 having a substantially rectangular bar-shape; the body augmenter 1 having a first end, a second end 14 in the opposite direction of the first end, and four sides 11; the body augmenter 1 having a recess 15 on the second end 14, and a screw hole 16 lengthwise formed on the second end 14 and communicating with the recess 15; each of the four sides 11 having a sloping portion 12 near to the first end; the four sides 11 of the body augmenter 1 having cavities 13 on other portions thereof, which cavities 13 communicate with each other, and form a hollow area together.
